# EUROPEAN PATENT APPLICATION

(11) **EP 4 261 839 A1**
(43) Date of publication of application: **18.10.2023**
(21) Application number: 21906784.0
(22) Date of filing: 25.08.2021
(51) Int. Cl.: G16H 30/40, G16H 50/50, G16H 50/20, G16H 50/30, A61B 6/00, A61B 6/03, A61B 5/08, A61B 5/00

(54) **LESION DIAGNOSIS METHOD**

(30) Priority: 14.12.2020 KR 20200174710
(71) Applicant: VUNO INC., Seoul 06541 (KR)
(72) Inventor: PARK, Beomhee, Seoul 05045 (KR); JUNG, Kyuhwan, Seoul 06599 (KR); KIM, Seyun, Hwaseong-si, Gyeonggi-do 18472 (KR)
(74) Representative: De Bonis, Paolo
(86) International application number: PCT/KR2021/011331
(87) International publication number: WO 2022/131479

(57) **Abstract**

A lesion diagnosing method includes inputting medical data into first and second neural network models to detect an object region for lesion diagnosis from the medical data, and detect at least one finding region related to a specific lesion; calculating a volume and location for at least one finding region included in the object region; and generating result information for the medical data based on a volume and a location for the finding region.

## Description

### [Technical Field]

The present invention relates to a lesion diagnosing method, and more particularly, to a method of diagnosing lesion information included in medical data.

### [Background Art]

In general, medical images greatly assist doctors in diagnosing patients by allowing the doctors to see a patient's internal body. For example, it is possible to confirm whether there is an abnormality in the heart, lungs, or bronchi through medical images.

However, some medical images are very difficult to diagnose, so that in some cases, even for medical staffs with years of experience, it may be difficult to make a quick judgment. Specifically, in the case of lung CT images, there are many types of nodules and the difficulty of diagnosing such nodules is quite high. Further, very minute abnormalities included in the medical image are likely to be overlooked when they are diagnosed by the human eye.

In recent years, an artificial intelligence model which performs the direct diagnosis from an input image was used, but this method may cause overfitting problems due to insufficient data or improperly collected data. Further, it is difficult to check which part was used by the artificial intelligence model to make a judgment, thus usability may be degraded.

Accordingly, there is a demand in the art to assist the doctors to analyze the medical image and diagnose the lesions.

Korean Unexamined Patent Publication Application No. 2019-0105461 discloses a computer assistance diagnosis system.

### [Disclosure]

### [Technical Problem]

The present disclosure has been made in response to the above-described background and an object thereof is to provide a lesion diagnosing method for diagnosis lesion information included in medical data.

### [Technical Solution]

In order to achieve the above-described objects, according to an exemplary embodiment of the present disclosure, a lesion diagnosing method includes inputting medical data into a first neural network model and a second neural network models to detect an object region for lesion diagnosis from the medical data, and to detect at least one finding region related to a specific lesion; calculating a volume and location for at least one finding region included in the object region; and generating result information for the medical data based on the volume and the location for the finding region.

In an alternative exemplary embodiment of the lesion diagnosing method, the detecting of the object region and at least one finding region related to the specific lesion for the lesion diagnosis includes: detecting the object region for lesion diagnosis from the medical data by inputting the medical data to the first neural network model; and detecting at least one finding region related to the specific lesion from the medical data by inputting the medical data to the second neural network model.

In an alternative exemplary embodiment of the lesion diagnosing method, in the detecting of an object region for lesion diagnosis from the medical data, the medical data includes a CT image and an object region for lesion diagnosis includes a lung and a lung lobe.

In an alternative exemplary embodiment of the lesion diagnosing method, the detecting of at least one finding region related to the specific lesion from the medical data includes: detecting a plurality of finding regions for lesions related to a respiratory disease from the medical data, and the plurality of finding regions includes a first finding region corresponding to ground glass opacity (GGO), a second finding region corresponding to consolidation, a third finding region corresponding to reticular opacity, a fourth finding region corresponding to pleural effusion, and a fifth finding region corresponding to emphysema.

In an alternative exemplary embodiment of the lesion diagnosing method, detecting the object region for lesion diagnosis from the medical data by inputting the medical data to a first neural network model; and detecting at least one finding region related to the specific lesion from the object region by inputting medical data including the object region detected through the first neural network model to a second sub neural network model are included.

In an alternative exemplary embodiment of the lesion diagnosing method, the calculating of the volume and the location for at least one finding region included in the object region for lesion diagnosis includes: calculating a volume for the object region; calculating a volume and a location for a finding region included in the object region; and calculating a relative volume ratio of the finding region to the object region.

In an alternative exemplary embodiment of the lesion diagnosing method, the calculating of a volume and a location for at least one finding region included in the object region for lesion diagnosis further includes: when there is a plurality of finding regions, calculating a total volume for the plurality of finding regions and a relative total volume ratio of the plurality of finding regions with respect to the object region.

In an alternative exemplary embodiment of the lesion diagnosing method, in the calculating of a volume and a location for at least one finding region included in the object region for lesion diagnosis includes: a distribution profile of the finding region is calculated based on the location of the finding region.

In an alternative exemplary embodiment of the lesion diagnosing method, in the generating of result information for the medical data based on the volume and the location for the finding region includes quantification data corresponding to the volume and the location for the finding region is input to a third neural network model to generate result information for the medical data.

In an alternative exemplary embodiment of the lesion diagnosing method, in the generating of result information for the medical data based on the volume and the location for the finding region includes, quantification data corresponding to the volume and the location for the finding region is input to a third neural network model to classify a class for the medical data.

In an alternative exemplary embodiment of the lesion diagnosing method, the class represents a class of the medical data related to a respiratory disease, and the class includes at least one of normal, abnormal, a mild case, a severe case, or a low risk group, a medium risk group, a high risk group corresponding to a treatment prognosis, or a type of a respiratory disease.

In an alternative exemplary embodiment of the lesion diagnosing method, in the generating of result information for the medical data based on the volume and the location for the finding region includes, when there is a plurality of finding regions, a respiratory disease prediction probability score included in the medical data is calculated based on a relative volume , an absolute volume, and a location of each finding region for a lung volume, and a relative volume, an absolute volume, and a location of each finding region for each lung lobe volume, and wherein each finding region is any one of a first finding region corresponding to ground glass opacity (GGO), a second finding region corresponding to consolidation, a third finding region corresponding to reticular opacity, a fourth finding region corresponding to pleural effusion, and a fifth finding region corresponding to emphysema.

In order to achieve the above-described objects, according to an exemplary embodiment of the present disclosure, a user terminal for lesion diagnosis includes: a processor including one or more cores; a memory; and an output unit providing a user interface. The user interface displays result information for the medical data in response to medical data input, and wherein the result information for the medical data is generated based on a result of calculating a volume and a location for at least one finding region included in an object region, based on the at least one finding region related to a specific lesion and the object region for lesion diagnosis detected from the medical data.

In the alternative exemplary embodiment of a user terminal for lesion diagnosis, the result information for the medical data includes at least one of a distribution image of the finding region included in the object region for lesion diagnosis, prediction probability information for respiratory disease, and summary information for the object region for lesion diagnosis and the finding region included in the object region.

In the alternative exemplary embodiment of a user terminal for lesion diagnosis, the user interface displays result information for the medical data in response to a user input, and the result information for the medical data is extracted from a database in which result information generated based on the volume and the location for at least one finding region included in the object region is stored.

In order to achieve the above-described objects, according to an exemplary embodiment of the present disclosure, a computer program stored in a computer readable storage medium is provided. When the computer program is executed by one or more processors, the computer program performs the following operations for lesion diagnosis, the operations including: an operation of inputting medical data into first and second neural network models to detect an object region for lesion diagnosis from the medical data, and detect at least one finding region related to a specific lesion; an operation of calculating a volume and location for at least one finding region included in the object region; and an operation of generating result information for the medical data based on a volume and a location for the finding region.

In order to achieve the above-described objects, according to an exemplary embodiment of the present disclosure, a computing device for providing a lesion diagnosing method, comprising: a processor including one or more cores; and a memory and the processor is configured to input medical data into a first neural network model and second neural network models to detect an object region for lesion diagnosis from the medical data, and to detect at least one finding region related to a specific lesion, calculate a volume and a location for at least one finding region included in the object region, and in the generating of result information for the medical data based on a volume and a location for the finding region,

### [Advantageous Effects]

The present invention provides a lesion diagnosing method for diagnosing lesion information included in medical data.

### [Description of Drawings]

In order to understand the above-mentioned features of the present disclosure in more detail and more specific with reference to the following exemplary embodiments, some of exemplary embodiments are illustrated in the accompanying drawings. Further, like reference numerals in the drawings are intended to refer to the same or similar functions through various aspects. However, it should be noted that the accompanying drawings merely illustrate specific typical exemplary embodiments of the present disclosure and are not to be considered as limiting the scope of the present invention, and that other embodiments having the same effect may be fully appreciated.
FIG. 1 is a view illustrating a block diagram of a computing device which performs an operation for providing a lesion diagnosing method according to an exemplary embodiment of the present disclosure.
FIG. 2 is a schematic view illustrating a network function according to an exemplary embodiment of the present disclosure.
FIG. 3 is a block diagram for explaining a lesion diagnosis process according to an exemplary embodiment of the present disclosure.
FIG. 4 is a block diagram for explaining a lesion diagnosis process according to another exemplary embodiment of the present disclosure.
FIG. 5 is a view for explaining a corona virus disease diagnosis process, according to an exemplary embodiment of the present disclosure.
FIGS. 6a to 6c are views for explaining a process of quantifying an object region and a finding region for lesion diagnosis, according to an exemplary embodiment of the present disclosure.
FIG. 7 is a view illustrating lesion diagnosis result information according to an exemplary embodiment of the present disclosure.
FIG. 8 is a flowchart for explaining a lesion diagnosis process according to an exemplary embodiment of the present disclosure.
FIG. 9 illustrates a simple and general schematic view of an exemplary computing environment in which exemplary embodiments of the present disclosure are embodied.

### [Best Mode]

Hereinafter, various embodiments are described with reference to the drawings. In the present specification, various descriptions are presented for understanding the present disclosure. However, it is obvious that the embodiments may be carried out even without a particular description.

Terms, "component", "module", "system", and the like used in the present specification indicate a computer-related entity, hardware, firmware, software, a combination of software and hardware, or execution of software. For example, a component may be a procedure executed in a processor, a processor, an object, an execution thread, a program, and/or a computer, but is not limited thereto. For example, both an application executed in a computing device and the computing device may be components. One or more components may reside within a processor and/or an execution thread. One component may be localized within one computer. One component may be distributed between two or more computers. Further, the components may be executed by various computer readable medium having various data structures stored therein. For example, components may communicate through local and/or remote processing according to a signal (for example, data transmitted to another system through a network, such as Internet, through data and/or a signal from one component interacting with another component in a local system and a distributed system) having one or more data packets.

A term "or" intends to mean comprehensive "or", not exclusive "or". That is, unless otherwise specified or when it is unclear in context, "X uses A or B" intends to mean one of the natural comprehensive substitutions. That is, when X uses A, X uses B, or X uses both A and B, "X uses A or B" may be applied to any one among the cases. Further, a term "and/or" used in the present specification shall be understood to designate and include all of the possible combinations of one or more items among the listed relevant items.

A term "include" and/or "including" shall be understood as meaning that a corresponding characteristic and/or a constituent element exists. Further, a term "include" and/or "including" means that a corresponding characteristic and/or a constituent element exists, but it shall be understood that the existence or an addition of one or more other characteristics, constituent elements, and/or a group thereof is not excluded. Further, unless otherwise specified or when it is unclear that a single form is indicated in context, the singular shall be construed to generally mean "one or more" in the present specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the embodiments disclosed herein may be additionally implemented as electronic hardware, computer software, or combinations of both sides. To clearly illustrate the interchangeability of hardware and software, various illustrative components, blocks, constitutions, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented embodiments is provided so that those skilled in the art of the present disclosure use or implement the present disclosure. Various modifications to the embodiments will be apparent to those skilled in the art. Generic principles defined herein may be applied to other embodiments without departing from the scope of the present disclosure. Therefore, the present disclosure is not limited to the embodiments presented herein. The present disclosure should be analyzed within the widest range which is coherent with the principles and new features presented herein.

In one exemplary embodiment of the present disclosure, the server may include other configurations for performing a server environment. The server may include any type of devices. The server may be digital equipment having an arithmetic capacity such as a laptop computer, a notebook computer, a desktop computer, a web pad, a mobile phone in which a processor is mounted and a memory is equipped. The server may be a web server which processes the service. The type of server described above is only an example and the present disclosure is not limited thereto.

In the present disclosure, a neural network, an artificial neural network, and a network function may be exchangeably used in some cases.

The term "image" or "image data" used throughout the detailed description and claims of the present invention refers to multi-dimensional data configured by discrete image elements (for example, pixels in the case of a two-dimensional image) and in other words, is a term referring to a visible object (for example, displayed on a video screen) or a digital representation of the object such as a file corresponding to a pixel output of a CT or an MRI detector.

For example, an image may be a medical image of a subject collected by a computed tomography (CT), magnetic resonance imaging (MRI), fundus images, ultrasonic wave or any other medical imaging system known in the art. However, the image does not necessarily have to be provided in a medical context, but may be provided in a non-medical context, and for example, may include X-ray imaging for security screening.

Throughout the detailed description and claims of the present invention, "digital imaging and communications in medicine (DICOM)" standard is a general term of various standards used for digital image expression and communication in medical devices and the DICOM standard is published by a joint committee formed by the American College of Radiology (ACR) and the National Electrical Manufactures Association (NEMA).

Further, throughout the detailed description and claims of the present invention, the picture archiving and communication system (PACS) is a term that refers to a system that stores, processes, and transmits according to the DICOM standard and in PACS, medical imaging images acquired using digital medical imaging equipment, such as X-ray, CT, or MRI are stored in the DICOM format and are transmitted to terminals inside and outside the hospital through the network and diagnosis results and medical records may be added thereto.

Throughout the specification, a neural network and a network function may be used as the same meaning. The neural network may generally be configured by a set of interconnected calculating units which may be referred to as "nodes". The "nodes" may also be referred to as "neurons". The neural network is configured to include at least two or more nodes. The nodes (or neurons) which configure the neural networks may be connected to each other by one or more "links".

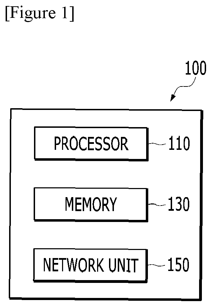

FIG. 1 is a view illustrating a block diagram of a computing device which performs an operation for providing a lesion diagnosing method according to an exemplary embodiment of the present disclosure.

A configuration of the computing device 100 illustrated in FIG. 1 is only an example simplified and illustrated. In an exemplary embodiment of the present disclosure, the computing device 100 may include other components for performing a computing environment of the computing device 100, and only some of the disclosed components may constitute the computing device 100.

The computing device 100 may include a processor 110, a memory 130, and a network unit 150.

In the present disclosure, the processor 110 relates to a method for diagnosis lesion information included in medical data to generate result information and inputs medical data including a chest image to first and second neural network models to detect an object region for lesion diagnosis from the medical data, detects at least one finding region related to a specific lesion, calculates a volume and a location of at least one finding region included in the object region, and generate result information for the medical data based on the volume and the location of the finding region.

The processor 110 diagnoses a specific lesion from the medical data to generate and display a lesion diagnosis result.

The object region defines a range to diagnose the lesion from the medical image and for example, in order to diagnose a lesion from the lung, a lung area is limited as the object region and in order to diagnose a lesion from a lung lobe of the lung, the lung lobe area may be limited as the object region.

The medical data may include at least one of image data, voice data, and time-series data. That is, an arbitrary type of data which allows a person engaged in the medical industry or a diagnostic device to determine the presence or absence of a disease in the data may be included in the medical data. The image data includes all image data which is output after imaging or measuring an affected part of the patient through examination equipment and converting the measured affected part into an electrical signal. The image data may include image data which configures each frame of a video, from a video continuously captured over time, from a medical imaging device. For example, the image data includes ultrasound examination image data, image data by MRI devices, CT tomography image data, and X-ray image data. Moreover, when voice data is converted into an electrical signal to be output as a graph type image or time-series data is represented as a visualized material, such as a graph, the image or the material may be included in the image data. For example, the medical data may include a CT image. The above-described example of the medical data is just an example, but does not limit to the present disclosure.

As an exemplary embodiment, when an object region for lesion diagnosis and at least one of finding regions related to a specific lesion are detected, the processor 110 inputs medical data to a first neural network model to detect an object region for lesion diagnosis from the medical data and inputs the medical data to a second neural network model to detect at least one of finding regions related to a specific lesion from the medical data. The medical data input to the first and second neural network models may be the same medical data. The first and second neural network models are disposed in parallel to perform the segmentation. The first and second neural network models may be the same neural network model. The first and second neural network models may be different neural network models. The first and second neural network models may be models for detecting an area for lesion diagnosis from the image to segment the area. The first and second neural network models may include a machine learning model including a neural network.

When an object region for lesion diagnosis and at least one finding region related to a specific lesion are detected, the processor 110 checks whether the object region for lesion diagnosis and at least one finding region related to a specific lesion overlap and removes a finding region which does not overlap the object region. When the processor 110 checks whether the object region for lesion diagnosis and at least one finding region related to a specific lesion overlap, the processor 110 calculates a location of the object region and a location of at least one finding region related to a specific lesion and checks whether the object region for lesion diagnosis and at least one finding region related to a specific lesion overlap based on the calculated locations of the object region and the finding region. Further, when the finding region which does not overlap the object region for lesion diagnosis is removed, the processor 110 removes the finding region located out of the object region to leave only the finding region located in the object region.

When the object region for lesion diagnosis is detected from the medical data, if there is a plurality of object regions, the processor 110 detects the object regions using different neural network models. For example, when the object region is detected from the medical data, the processor 110 detects a lung area for lesion diagnosis from medical data using a first sub neural network model included in a first neural network model and detects a plurality of lung lobes from the detected lung area using a second sub neural network model included in the first neural network model. When the lung area for lesion diagnosis is detected from the medical data, the processor 110 detects the lung area from the medical data using a 2D segmentation model. For example, the 2D segmentation model may be a 2D U-Net, but this is merely an example, but the present disclosure is not limited thereto. Further, when a plurality of lung lobes is detected from the detected lung area, the processor 110 detects the plurality of lung lobes from the lung area detected using a 3D segmentation model. For example, the 3D segmentation model may be a 3D U-Net, but this is merely an example, but the present disclosure is not limited thereto. The reason why different models are used to detect the lung and the lung lobe is because a 3D segmentation model which has a more computational amount than the 2D segmentation model is used only for the segmentation of the lung lobe to increase the computational efficiency and enable precise segmentation.

When the processor 110 detects the object region for lesion diagnosis from the medical data, the medical data includes a CT image and the object region includes the lung and the lung lobe. The processor 110 may detect the lung area for lesion diagnosis from the medical data and detects a plurality of lung lobe areas from the detected lung area. The plurality of lung lobe areas includes a right upper lung lobe (RUL), a right middle lung lobe (RML), a right lower lung lobe (RLL), a left upper lung lobe (LUL), and a left lower lung lobe (LLL). When the object region for lesion diagnosis includes a lung and a lung lobe, a neural network model used to detect the lung area for lesion diagnosing and a neural network model used to detect the lung lobe area for lesion diagnosing are different in the processor 110. For example, in the processor 110, the neural network model used to detect the lung area for lesion diagnosis includes a 2D segmentation model and the neural network model used to detect the lung lobe area for lesion diagnosis may include a 3D segmentation model. However, this is merely an example, but the present disclosure is not limited thereto. The reason why different models are used to detect the lung and the lung lobe is because a 3D segmentation model which has a more computational amount than the 2D segmentation model is used only for the detection of the lung lobe to increase the computational efficiency and enable precise segmentation.

When at least one finding region related to a specific lesion is detected from the medical data, the processor 110 detects at least one finding region related to a specific lesion, among a plurality of finding regions corresponding to ground glass opacity (GGO), consolidation, reticular opacity, pleural effusion, emphysema, and normal, from the medical data. For example, the finding region corresponding to the ground glass opacity (GGO) refers to an area which in the medical data which is a CT image, if there is partial filling of the alveolar cavity or slight thickening of the alveolar wall, does not appear as an independent object shade, but appear as a blurry shade. The finding region corresponding to consolidation refers to an area which appears as an opaque shade when the lung is filled with exudate, pus, blood, or other cells or substances instead of air in the medical data which is a CT image. The finding region corresponding to reticular opacity refers to an area which appears as reticular opacity when the tissues constituting the lung interstitium are thickened by liquid material, fibrotic tissue, or cells in the medical data which is a CT image. The finding region corresponding to pleural effusion refers to an area showing liquid abnormality accumulated in the pleural cavity in the medical data which is a CT image. The finding region corresponding to emphysema refers to an area in which the alveoli expand due to the loss of elasticity due to the destruction of the wall between the alveoli, overextended air and the diaphragm appear as a flat shade in the medical data which is a CT image. However, this is merely an example, but the present disclosure is not limited thereto.

When at least one finding region related to a specific lesion is detected from the medical data, the processor 110 may detect a first finding region corresponding to the ground glass opacity (GGO) related to a respiratory disease lesion from the medical data, a second finding region corresponding to consolidation, a third finding region corresponding to reticular opacity, a fourth finding region corresponding to pleural effusion, and a fifth finding region corresponding to emphysema. For example, the respiratory disease may include a COVID-19 related virus. However, this is merely an example, but the present disclosure is not limited thereto.

When at least one finding region related to a specific lesion is detected from the medical data, the processor 110 detects at least one finding region related to a specific lesion from medical data using a second neural network model including a 2D segmentation model or a 3D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto.

When at least one finding region related to a specific lesion is detected from the medical data, the processor 110 detects an infected area related to the specific lesion from the medical data using the 3D segmentation model and detects at least one finding region from the detected infected area using the 2D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto. The reason why different models are used to detect the infected area and the finding region is because a 3D segmentation model which has a more computational amount than the 2D segmentation model is used only for the detection of the infected area to increase the computational efficiency and enable precise segmentation.

As another exemplary embodiment, when an object region for lesion diagnosis and at least one of finding regions related to a specific lesion is detected, the processor 110 inputs medical data to a first neural network model to detect an object region from the medical data and inputs the medical data corresponding to the detected object region to a second neural network model to detect at least one finding region related to a specific lesion from the object region. The first and second neural network models are disposed in series to perform the segmentation. The first and second neural network models may be the same neural network model. The first and second neural network models may be different neural network models. The first and second neural network models may be models for detecting an area for lesion diagnosis from the image to segment the area. The first and second neural network models may include a machine learning model including a neural network. When the obj ect region for lesion diagnosis is detected from the medical data, if there is a plurality of object regions for lesion diagnosis, the processor 110 detects the object regions for lesion diagnosis using different neural network models. For example, when the object region for lesion diagnosis is detected from the medical data, the processor 110 detects a lung area for lesion diagnosis from medical data using a first sub neural network model included in a first neural network model and detects a plurality of lung lobes from the detected lung area using a second sub neural network model included in the first neural network model. When the lung area for lesion diagnosis is detected from the medical data, the processor 110 detects the lung area for lesion diagnosis from the medical data using a 2D segmentation model. For example, the 2D segmentation model may be a 2D U-Net, but this is merely an example, but the present disclosure is not limited thereto. Further, when a plurality of lung lobes is detected from the detected lung area, the processor 110 detects the plurality of lung lobes from the segmented lung area using a 3D segmentation model. For example, the 3D segmentation model may be a 3D U-Net, but this is merely an example, but the present disclosure is not limited thereto. The reason why different models are used to detect the lung and the lung lobe is because a 3D segmentation model which has a more computational amount than the 2D segmentation model is used only for the detection of the lung lobe to increase the computational efficiency and enable precise segmentation.

When at least one finding region related to a specific lesion is detected from the medical data, the processor 110 detects at least one finding region related to a specific lesion from medical data using a second neural network model including a 2D segmentation model or a 3D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto.

When at least one finding region related to a specific lesion is detected from the medical data, the processor 110 detects an infected area related to the specific lesion from the medical data using the 3D segmentation model and detects at least one finding region from the detected infected area using the 2D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto. The reason why different models are used to detect the infected area and the finding region is because a 3D segmentation model which has a more computational amount than the 2D segmentation model is used only for the detection of the infected area to increase the computational efficiency and enable precise segmentation.

When a volume and a location for at least one finding region included in the object region for lesion diagnosis are calculated, the processor 110 calculates a volume for the object region and calculates a volume and a location for the finding region included in the object region, and calculates a relative volume ratio of the finding region for the object region. When there is a plurality of finding regions, the processor 110 calculates a total volume for the plurality of finding regions and a relative total volume ratio of the plurality of finding regions with respect to the object region. For example, when the volume and the location for at least one finding region included in the object region for lesion diagnosis are calculated, if the object region for lesion diagnosis includes a plurality of lungs and a plurality of lung lobes, the processor 110 calculates a volume and a location for at least one finding region included in each lung and calculates a volume and a location for at least one finding region included in each lung lobe.

The processor 110 calculates a distribution profile of the finding region based on the location of the finding region when the volume and the location for at least one finding region included in the object region for lesion diagnosis are calculated. For example, the distribution profile of the finding region includes at least any one of bilateral distribution of the finding region, peripheral distribution, posterior distribution, and basal distribution.

When result information about the medical data is generated based on the volume and the location of the finding region, the processor 110 inputs quantification data corresponding to the volume and the location of the finding region to the third neural network model to generate result information for the medical data. The third neural network model may be a model which classifies and predicts a class for medical data. For example, the third neural network model includes a random forest model. The third neural network model may include a machine learning model including a neural network. The processor 110 inputs the quantification data corresponding to the volume and the location of the finding region to the third neural network model to classify the class for the medical data. For example, the class indicates a class of the medical data related to the respiratory disease and the class may include at least one of normal, abnormal, mild, severe, or a high risk group, a medium risk group, a low risk group, or a type of respiratory disease corresponding to a treatment prognosis. When there is a plurality of finding regions, the processor 110 calculates a respiratory disease prediction probability score included in the medical data based on the relative volume and the location of each finding region for lung volume and the relative volume and the location of each finding region for each lung lobe volume. Each finding region may be any one of a first finding region corresponding to the ground glass opacity (GGO), a second finding region corresponding to consolidation, a third finding region corresponding to the reticular opacity, a fourth finding region corresponding to the pleural effusion, and a fifth finding region corresponding to emphysema.

When result information about the medical data is generated based on the volume and the location of the finding region, the processor 110 inputs quantification data corresponding to the volume and the location of the finding region to the trained third neural network model to diagnose and predict the respiratory disease. When there is a plurality of object regions for lesion diagnosis, the processor 110 inputs the quantification data including a relative volume of each finding region for each object region, an absolute volume, a distribution profile of each finding region, and a number of object regions in which the finding region is distributed based on a distribution profile of each finding region to the trained third neural network model to diagnose and predict the respiratory disease. Further, the processor 110 inputs the quantification data corresponding to the volume and the location of the finding region to the trained third neural network model to classify the respiratory disease and normal. Further, the processor 110 inputs the quantification data corresponding to the volume and the location for the finding region to the trained third neural network model to classify the respiratory disease and the other disease. Further, the processor 110 inputs the quantification data corresponding to the volume and the location of the finding region to the trained third neural network model to classify a severe case and a mild case for the respiratory disease. Further, the processor 110 inputs the quantification data corresponding to the volume and the location of the finding region to the trained third neural network model to classify the treatment prognosis for the respiratory disease into a high risk group, a medium risk group, and a low risk group.

As another exemplary embodiment, the processor 110 inputs the medical data to the first neural network model to detect an object region for lesion diagnosis from the medical data, inputs the medical data to the second neural network model to detect at least one finding region related to a specific lesion from the medical data, and inputs the medical data to the fourth neural network model to detect at least one finding region related to a COVID-19 lesion from the medical data. The medical data input to the first, second, and fourth neural network models may be the same medical data. The first, second, and fourth neural network models are disposed in parallel to perform the segmentation. The first, second, and fourth neural network models may be the same neural network model. The first, second, and fourth neural network models may be different neural network models. The first, second, and fourth neural network models may be models for detecting an area for lesion diagnosis from the image to segment the area. The first, second, and fourth neural network models may include a machine learning model including a neural network. The processor 110 calculates a volume of the object region detected through the first neural network model and calculates a volume and a location for the finding region detected through the second and fourth neural network models, and calculates a relative volume ratio of the finding region for the object region. The processor 110 inputs the quantification data corresponding to the volume and the location of the finding region to the trained third neural network model to diagnose and predict the respiratory disease.

As another exemplary embodiment, the processor 110 inputs the medical data to the first neural network model to detect an object region for lesion diagnosis from the medical data, inputs the detected object region to the second neural network model to detect at least one finding region related to a specific lesion from the object region, and inputs the detected finding region to the fourth neural network model to detect a finding region related to a COVID-19 lesion from the finding region. The first, second, and fourth neural network models are disposed in series to perform the segmentation. The first, second, and fourth neural network models may be the same neural network model. The first, second, and fourth neural network models may be different neural network models. The first, second, and fourth neural network models may be models for detecting an area for lesion diagnosis from the image to segment the area. The first, second, and fourth neural network models may include a machine learning model including a neural network. The processor 110 calculates a volume of the object region detected through the first neural network model and calculates a volume and a location for the finding region detected through the second and fourth neural network models, and calculates a relative volume ratio of the finding region for the object region. The processor 110 inputs the quantification data corresponding to the volume and the location of the finding region to the trained third neural network model to diagnose and predict the respiratory disease.

The above-described previously trained neural network model may be a deep neural network. Throughout the specification, a neural network, a network function, and a neural network may be used to have the same meaning. A deep neural network (DNN) may refer to a neural network including a plurality of hidden layers in addition to the input layer and the output layer. When the deep neural network is used, latent structures of the data may be figured out. That is, it is possible to identify latent structures of photos, texts, video, audio, and music (for example, which objects are in the photo, what is the content and the emotion of the text, and what is the content and the emotion of the audio). The deep neural network may include a convolutional neural network (CNN), a recurrent neural network (RNN), a restricted boltzmann machine (RBM), a deep belief network (DBN), a Q network, a U network, and a Siamese network.

The convolutional neural network is a kind of a deep neural network and includes a neural network including a convolutional layer. The convolutional neural network is one kind of multilayer perceptrons designed to use minimum preprocess. The CNN may be configured by one or several convolutional layers and artificial neural network layers coupled thereto. The CNN may additionally utilize a weight and pooling layers. With this structure, the CNN may sufficiently utilize input data with a 2D structure. The convolutional neural network may be used to recognize an object from the image. The convolutional neural network represents and processes the image data as a matrix with a dimension. For example, in the case of image data encoded with red-green-blue RGB, the image data may be represented as a 2D matrix (for example, when the image is a 2D image) for every color of R, G, and B. That is, a color value of each pixel of the image data may be a component of the matrix and the magnitude of the matrix may be equal to the image size. Accordingly, the image data may be represented with three 2D matrixes (3D data arrays).

In the convolutional neural network, the convolutional process (input or output a convolutional layer) is performed by multiplying a convolutional filter and the matrix component in each location of the image while moving the convolutional filter. The convolutional filter may be configured by a n*n matrix. The convolutional filter may be configured by a fixed filter which is smaller than a number of total pixels of the image. That is, when the m*m image is input to the convolutional layer (for example, a convolutional layer in which a size of the convolutional filter is n*n), a matrix representing n*n pixels including each pixel of the image may be a product of the convolutional filter and the component (that is, a product of each component of the matrix). A component matching the convolutional filter may be extracted from the image by the product with the convolutional filter. For example, a 3*3 convolutional filter for extracting a vertical linear component from the image may be configured by [[0, 1, 0], [0, 1, 0], [0, 1, 0]]. When the 3*3 convolutional filter for extracting a vertical linear component from the image is applied to the input image, a vertical linear component matching the convolutional filter is extracted from the image to be output. The convolutional layer may apply the convolutional filter to each matrix (that is, in the case of coding images R, G, and B, R, G, and B color) for each channel representing the image. The convolutional layer may apply the convolutional filter to the input image to extract a feature matching the convolutional filter from the input image. The filter value (that is, a value of each component of the matrix) of the convolutional filter may be updated by the backpropagation during the learning process of the convolutional neural network.

An output of the convolutional layer is connected to a sub sampling layer to simplify the output of the convolutional layer, to reduce a memory usage amount and a computational amount. For example, an output of the convolutional layer is input to a pooling layer having a 2*2 max pooling filter, a maximum value including each patch is output at every 2*2 patch in each pixel of the image to compress the image. The above-described pooling may be a method of outputting a minimum value from the patch or outputting a mean value of the patch, and an arbitrary pooling method may be included in the present disclosure.

The convolutional neural network may include one or more convolutional layers and a sub sampling layer. The convolutional neural network iteratively performs the convolutional process and the sub sampling process (for example, the above-described max pooling) to extract a feature from the image. The neural network may extract a global feature of the image through iterative convolutional process and sub sampling process.

An output of the convolutional layer or the sub sampling layer may be input to a fully connected layer. The fully connected layer is a layer in which all neurons in one layer and all neurons in an adjacent layer are connected. The fully connected layer may refer to a structure in which all nodes of each layer is connected to all nodes of the other layer in the neural network.

In the exemplary embodiment of the present disclosure, in order to perform segmentation for the medical data, the neural network includes a deconvolutional neural network (DCNN). The deconvolutional neural network performs a similar operation to a convolutional neural network computed backwards. The deconvolutional neural network outputs a feature extracted from the convolutional neural network as a feature map related to original data.

According to an exemplary embodiment, the processor 110 may be constituted by one or more cores, and include processors for data analysis and deep learning, such as a central processing unit (CPU), a general purpose graphics processing unit (GPGPU), a tensor processing unit (TPU), etc., of the computing device. The processor 110 may read a computer program stored in the memory 130 and process data for machine learning according to an exemplary embodiment of the present disclosure. According to an exemplary embodiment of the present disclosure, the processor 110 may perform an operation for learning the neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, the GPGPU, and the TPU of the processor 110 may process learning of the network function. For example, the CPU and the GPGPU may process the learning of the network function and data classification using the network function jointly. In addition, in an exemplary embodiment of the present disclosure, the learning of the network function and the data classification using the network function may be processed by using processors of a plurality of computing devices together. In addition, the computer program performed by the computing device according to an exemplary embodiment of the present disclosure may be a CPU, GPGPU, or TPU executable program.

According to an exemplary embodiment of the present disclosure, the memory 130 may store a computer program for performing lesion diagnosing and providing lesion diagnosis results, and the stored computer program may be read and executed by the processor 110. The memory 130 may store any type of information generated or determined by the processor 110 and any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

A network unit 150 according to an exemplary embodiment of the present disclosure transmits and receives specific lesion diagnosis result information to and from the other computing device or server. Further, the network unit 150 enables the communication between the plurality of computing devices so that operations for diagnosing a lesion or training a model can be performed in a distributed manner in each of the plurality of computing devices. The network unit 150 enables the communication between the plurality of computing devices to perform the operation for diagnosing a lesion or training a model using a network function in a distributed manner.

The network unit 150 according to the exemplary embodiment of the present disclosure may operate based on an arbitrary type of wired or wireless communication technology currently used and implemented, such as near range (short-distance), longdistance, wired, and wireless communication techniques, and may be used in other networks.

The computing device 100 of the present disclosure further includes an output unit and an input unit.

An output unit according to an exemplary embodiment of the present disclosure displays a user interface UI for providing a lesion diagnosis result. The output unit outputs an arbitrary type of information which is generated or determined by the processor 110 and an arbitrary type of information received by the network unit 150.

In the exemplary embodiment of the present disclosure, the output unit may include at least one of a liquid crystal display (LCD), a thin film transistor liquid crystal display (TFT LCD), an organic light emitting diode (OLED), a flexible display, and a 3D display. Among them, some display module may be configured to be transparent or light-transmissive to see the outside therethrough. This may be referred to as a transparent display module and a representative example of the transparent display module is a transparent OLED (TOLED).

The input unit according to the exemplary embodiment of the present disclosure receives a user input. The input unit may include keys and/or buttons on the user interface to receive a user input or physical keys and/or buttons. A computer program for controlling a display according to exemplary embodiments of the present disclosure may be executed according to the user input through the input unit.

The input unit according to the exemplary embodiments of the present disclosure senses button manipulation or touch input of the user to receive a signal or receives a voice or an operation of the user through a camera or a microphone to convert the voice or the operation into an input signal. To this end, speech recognition techniques or motion recognition techniques may be used.

The input unit according to the exemplary embodiments of the present disclosure may be implemented as external input equipment connected to the computing device 100. For example, the input equipment may be at least one of a touch pad, a touch pen, a keyboard, and a mouse to receive the user input, but this is just illustrative and the present disclosure is not limited thereto.

The input unit according to the exemplary embodiment of the present disclosure recognizes a user touch input. The input unit according to the exemplary embodiment of the present disclosure may be the same configuration as the output unit. The input unit may be configured as a touch screen which is implemented to receive a selection input of the user. The touch screen may use any one of a contact capacitive method, an infrared light sensing method, a surface acoustic wave (SAW) method, a piezoelectric method, and a resistive film method. The above-detailed description of the touch screen is just an example according to an exemplary embodiment of the present disclosure and various touch screen panels may be employed for the computing device 100. The input unit configured by the touch screen includes a touch sensor. The touch sensor may be configured to convert a change of a pressure which is applied to a specific portion of the input unit or a capacity which is generated in a specific portion of the input unit into an electrical input signal. The touch sensor may be configured to detect not only a touched position and area, but also a pressure at the time of touching. When there is a touch input to the touch sensor, corresponding signal(s) is(are) sent to a touch controller. The touch controller processes the signal(s) and then transmits corresponding data to the processor 110. By doing this, the processor 110 may recognize which area of the input unit is touched.

In one exemplary embodiment of the present disclosure, the server may include other configurations for performing a server environment of the server. The server may include any type of devices. The server may be digital equipment having an arithmetic capacity such as a laptop computer, a notebook computer, a desktop computer, a web pad, and a mobile phone in which a processor is mounted and a memory is equipped.

A server (not illustrated) which performs an operation of providing a user interface which displays a lesion diagnosis result according to the exemplary embodiment of the present disclosure to the user terminal includes a network unit, a processor, and a memory.

The server generates the user interface according to the exemplary embodiments of the present disclosure. The server may be a computing system which provides information to a client (for example, a user terminal) through a network. The server transmits the generated user interface to the user terminal. In this case, the user terminal may be an arbitrary type of computing device 100 which is accessible to the server. The processor of the server may transmit the user interface to the user terminal through the network unit. The server according to the exemplary embodiments of the present disclosure may be a cloud server, for example. The server may be a web server which processes the service. The type of server described above is only an example and the present disclosure is not limited thereto.

Accordingly, according to the present disclosure, a respiratory disease is diagnosed from the medical data to increase a prediction probability for the respiratory disease. For example, a prediction reliability related to the respiratory disease, such as corona virus including COVID-19 may be improved.

Further, the present disclosure may provide user's convenience by visualizing the finding region for the respiratory disease diagnosed from the medical data and quantitatively displaying the volume and the location of the finding region. Further, the present disclosure assists in polymerase chain reaction (PCR) test, assists in physician diagnosis, or quantifies the severity and progression of a disease.

In the related art, an end-to-end model which directly diagnoses the COVID-19 from the input image has been used. However, this method may cause overfitting problems due to insufficient data or improperly collected data. Further, it is difficult to check which part was used by the artificial intelligence model to make a judgment, the usability may be degraded.

However, according to the present disclosure, a first neural network model which detects an object region and a second neural network model which detects a finding region are disposed in parallel or series to detect the object region and the finding region from the medical image. Further, volumes of the detected object region and the finding region are calculated to classify and predict a class for the medical image through the third neural network model based on the volume and the location of the finding region and generate the result information.

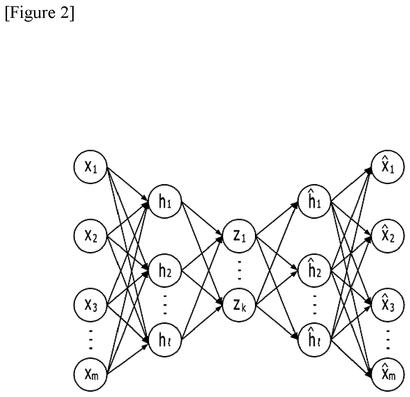

FIG. 2 is a schematic diagram illustrating a network function according to the embodiment of the present disclosure.

Throughout the present specification, the meanings of a calculation model, a nerve network, the network function, and the neural network may be interchangeably used. The neural network may be formed of a set of interconnected calculation units which are generally referred to as "nodes". The "nodes" may also be called "neurons". The neural network consists of one or more nodes. The nodes (or neurons) configuring the neural network may be interconnected by one or more links.

In the neural network, one or more nodes connected through the links may relatively form a relationship of an input node and an output node. The concept of the input node is relative to the concept of the output node, and a predetermined node having an output node relationship with respect to one node may have an input node relationship in a relationship with another node, and a reverse relationship is also available. As described above, the relationship between the input node and the output node may be generated based on the link. One or more output nodes may be connected to one input node through a link, and a reverse case may also be valid.

In the relationship between an input node and an output node connected through one link, a value of the output node data may be determined based on data input to the input node. Herein, a link connecting the input node and the output node may have a weight. The weight is variable, and in order for the neural network to perform a desired function, the weight may be varied by a user or an algorithm. For example, when one or more input nodes are connected to one output node by links, respectively, a value of the output node may be determined based on values input to the input nodes connected to the output node and weights set in the link corresponding to each of the input nodes.

As described above, in the neural network, one or more nodes are connected with each other through one or more links to form a relationship of an input node and an output node in the neural network. A characteristic of the neural network may be determined according to the number of nodes and links in the neural network, a correlation between the nodes and the links, and a value of the weight assigned to each of the links. For example, when there are two neural networks in which the numbers of nodes and links are the same and the weight values between the links are different, the two neural networks may be recognized to be different from each other.

The neural network may consist of a set of one or more nodes. A subset of the nodes configuring the neural network may form a layer. Some of the nodes configuring the neural network may form one layer on the basis of distances from an initial input node. For example, a set of nodes having a distance of n from an initial input node may form n layers. The distance from the initial input node may be defined by the minimum number of links, which need to be passed to reach a corresponding node from the initial input node. However, the definition of the layer is arbitrary for the description, and a degree of the layer in the neural network may be defined by a different method from the foregoing method. For example, the layers of the nodes may be defined by a distance from a final output node.

The initial input node may mean one or more nodes to which data is directly input without passing through a link in a relationship with other nodes among the nodes in the neural network. Otherwise, the initial input node may mean nodes which do not have other input nodes connected through the links in a relationship between the nodes based on the link in the neural network. Similarly, the final output node may mean one or more nodes that do not have an output node in a relationship with other nodes among the nodes in the neural network. Further, the hidden node may mean nodes configuring the neural network, not the initial input node and the final output node.

In the neural network according to the embodiment of the present disclosure, the number of nodes of the input layer may be the same as the number of nodes of the output layer, and the neural network may be in the form that the number of nodes decreases and then increases again from the input layer to the hidden layer. Further, in the neural network according to another embodiment of the present disclosure, the number of nodes of the input layer may be smaller than the number of nodes of the output layer, and the neural network may be in the form that the number of nodes decreases from the input layer to the hidden layer. Further, in the neural network according to another embodiment of the present disclosure, the number of nodes of the input layer may be larger than the number of nodes of the output layer, and the neural network may be in the form that the number of nodes increases from the input layer to the hidden layer. The neural network according to another embodiment of the present disclosure may be the neural network in the form in which the foregoing neural networks are combined.

A deep neural network (DNN) may mean the neural network including a plurality of hidden layers, in addition to an input layer and an output layer. When the DNN is used, it is possible to recognize a latent structure of data. That is, it is possible to recognize latent structures of photos, texts, videos, voice, and music (for example, what objects are in the photos, what the content and emotions of the texts are, and what the content and emotions of the voice are). The DNN may include a convolutional neural network (CNN), a recurrent neural network (RNN), an auto encoder, Generative Adversarial Networks (GAN), a Long Short-Term Memory (LSTM), a transformer, a restricted Boltzmann machine (RBM), a deep belief network (DBN), a Q network, a U network, a Siamese network, a Generative Adversarial Network (GAN), and the like. The foregoing description of the deep neural network is merely illustrative, and the present disclosure is not limited thereto.

In the embodiment of the present disclosure, the network function may include an auto encoder. The auto encoder may be one type of artificial neural network for outputting output data similar to input data. The auto encoder may include at least one hidden layer, and the odd-numbered hidden layers may be disposed between the input/output layers. The number of nodes of each layer may decrease from the number of nodes of the input layer to an intermediate layer called a bottleneck layer (encoding), and then be expanded symmetrically with the decrease from the bottleneck layer to the output layer (symmetric with the input layer). The auto encoder may perform a nonlinear dimension reduction. The number of input layers and the number of output layers may correspond to the dimensions after preprocessing of the input data. In the auto encoder structure, the number of nodes of the hidden layer included in the encoder decreases as a distance from the input layer increases. When the number of nodes of the bottleneck layer (the layer having the smallest number of nodes located between the encoder and the decoder) is too small, the sufficient amount of information may not be transmitted, so that the number of nodes of the bottleneck layer may be maintained in a specific number or more (for example, a half or more of the number of nodes of the input layer and the like).

The neural network may be trained by at least one scheme of supervised learning, unsupervised learning, semi-supervised learning, and reinforcement learning. The training of the neural network may be a process of applying knowledge for the neural network to perform a specific operation to the neural network.

The neural network may be trained in a direction of minimizing an error of an output. In the training of the neural network, training data is repeatedly input to the neural network and an error of an output of the neural network for the training data and a target is calculated, and the error of the neural network is back-propagated in a direction from an output layer to an input layer of the neural network in order to decrease the error, and a weight of each node of the neural network is updated. In the case of the supervised learning, training data labelled with a correct answer (that is, labelled training data) is used, in each training data, and in the case of the unsupervised learning, a correct answer may not be labelled to each training data. That is, for example, the training data in the supervised learning for data classification may be data, in which category is labelled to each of the training data. The labelled training data is input to the neural network and the output (category) of the neural network is compared with the label of the training data to calculate an error. For another example, in the case of the unsupervised learning related to the data classification, training data that is the input is compared with an output of the neural network, so that an error may be calculated. The calculated error is back-propagated in a reverse direction (that is, the direction from the output layer to the input layer) in the neural network, and a connection weight of each of the nodes of the layers of the neural network may be updated according to the backpropagation. A change amount of the updated connection weight of each node may be determined according to a learning rate. The calculation of the neural network for the input data and the backpropagation of the error may configure a learning epoch. The learning rate is differently applicable according to the number of times of repetition of the learning epoch of the neural network. For example, at the initial stage of the learning of the neural network, a high learning rate is used to make the neural network rapidly secure performance of a predetermined level and improve efficiency, and at the latter stage of the learning, a low learning rate is used to improve accuracy.

In the training of the neural network, the training data may be generally a subset of actual data (that is, data to be processed by using the trained neural network), and thus an error for the training data is decreased, but there may exist a learning epoch, in which an error for the actual data is increased. Overfitting is a phenomenon, in which the neural network excessively learns training data, so that an error for actual data is increased. For example, a phenomenon, in which the neural network learning a cat while seeing a yellow cat cannot recognize cats, other than a yellow cat, as cats, is a sort of overfitting. Overfitting may act as a reason of increasing an error of a machine learning algorithm. In order to prevent overfitting, various optimizing methods may be used. In order to prevent overfitting, a method of increasing training data, a regularization method, a dropout method of inactivating a part of nodes of the network during the training process, a method using a bath normalization layer, and the like may be applied.

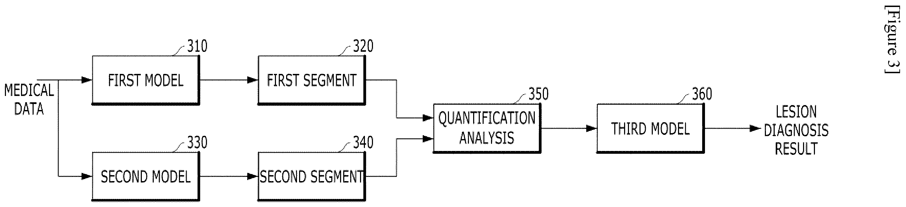

FIG. 3 is a block diagram for explaining a lesion diagnosis process according to an exemplary embodiment of the present disclosure.

As illustrated in FIG. 3, a computing device of the present disclosure segments an object region for lesion diagnosis from the medical data by inputting medical data to the first neural network model 310 to output a first segment 320. The computing device segments at least one finding region related to a specific lesion from the medical data by inputting the medical data to the second neural network model 330 to output a second segment 340. Here, the medical data input to the first and second neural network models 310 and 330 may be the same medical data. The first and second neural network models 310 and 330 may be models for detecting an area for lesion diagnosis from the image to segment the area.

When an object region for lesion diagnosis and at least one finding region related to a specific lesion are segmented, the computing device checks whether the object region for lesion diagnosis and at least one finding region related to a specific lesion overlap and removes a finding region which does not overlap the object region. When the computing device checks whether the object region for lesion diagnosis and at least one finding region related to a specific lesion overlap, calculates a location of the object region and a location of at least one finding region related to a specific lesion and checks whether the object region for lesion diagnosis and at least one finding region related to a specific lesion overlap based on the calculated locations of the object region and the finding region. Further, when the finding region which does not overlap the object region for lesion diagnosis is removed, the computing device removes the finding region located out of the object region to remain only the finding region located in the object region.

When the object region for lesion diagnosis includes a lung and a lung lobe, a neural network model used to segment the lung area for lesion diagnosing and a neural network model used to segment the lung lobe area for lesion diagnosing are different in the computing device. For example, in the computing device, the neural network model used to segment the lung area for lesion diagnosis includes a 2D segmentation model and the neural network model used to segment the lung lobe area for lesion diagnosis may include a 3D segmentation model. However, this is merely an example, but the present disclosure is not limited thereto. The reason why different models are used to segment the lung and the lung lobe is because a 3D segmentation model which has a more computational amount than the 2D segmentation model is used only for the segmentation of the lung lobe to increase the computational efficiency and enable precise segmentation.

When at least one finding region related to a specific lesion is segmented from the medical data, the computing device segments at least one finding region related to a specific lesion, among a plurality of finding regions corresponding to ground glass opacity (GGO), consolidation, reticular opacity, pleural effusion, emphysema, and normal, from the medical data. When at least one finding region related to a specific lesion is segmented from the medical data, the computing device may segment a first finding region corresponding to the ground glass opacity (GGO) related to a lesion of the corona virus disease, a second finding region corresponding to consolidation, a third finding region corresponding to reticular opacity, a fourth finding region corresponding to pleural effusion, and a fifth finding region corresponding to emphysema, from the medical data.

When at least one finding region related to a specific lesion is segmented from the medical data, the computing device segments at least one finding region related to a specific lesion from medical data using a second neural network model including a 2D segmentation model or a 3D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto.

The computing device performs a quantification analysis 350 for the finding region included in the object region by calculating a volume and a location for at least one finding region included in the object region for lesion diagnosis. When a volume and a location for at least one finding region included in the object region are calculated, the computing device calculates a volume for the object region and calculates a volume and a location for the finding region included in the object region, and calculates a relative volume ratio of the finding region for the object region. When there is a plurality of finding regions, the computing device calculates a total volume for the plurality of finding regions and a relative total volume ratio of the plurality of finding regions with respect to the object region.

The computing device diagnoses and predicts the respiratory disease by inputting quantification data corresponding to the volume and the location for the finding region calculated through the quantification analysis 350 to the previously trained third neural network model 360. The third neural network model 360 may be a model which classifies and predicts a class for medical data. For example, the third neural network model 360 includes a random forest model. The third neural network model 360 may include a machine learning model including a neural network. The computing device diagnoses the respiratory disease from the medical data to generate and display the result information. When there is a plurality of object regions for lesion diagnosis, the computing device inputs the quantification data including a relative volume of each finding region for each object region, a distribution profile of each finding region, and a number of object regions in which the finding region is distributed based on a distribution profile of each finding region to the trained third neural network model 360 to diagnose and predict the respiratory disease. Further, the computing device inputs the quantification data corresponding to the volume and the location of the finding region to the trained third neural network model to classify the respiratory disease and normal. Further, the computing device inputs the quantification data corresponding to the volume and the location of the finding region to the trained third neural network model to classify the respiratory disease and the other disease. Further, the computing device inputs the quantification data corresponding to the volume and the location of the finding region to the trained third neural network model to classify a severe case and a mild case for the respiratory disease. Further, the computing device inputs the quantification data corresponding to the volume and the location of the finding region to the trained third neural network model to classify the treatment prognosis for the respiratory disease into a high risk group, a medium risk group, and a low risk group.

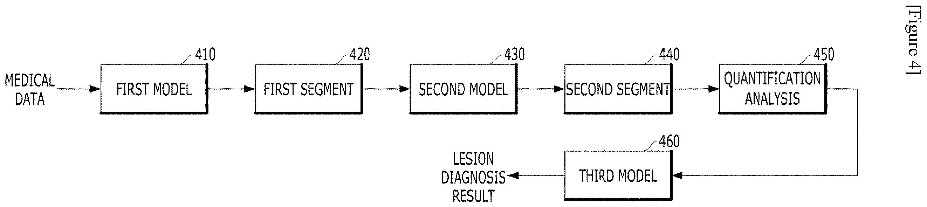

FIG. 4 is a block diagram for explaining a lesion diagnosis process according to another exemplary embodiment of the present disclosure.

As illustrated in FIG. 4, a computing device of the present disclosure segments an object region for lesion diagnosis from the medical data by inputting medical data to the first neural network model 410 to output a first segment 420. The computing device segments at least one finding region related to a specific lesion from the medical data by inputting a first segment 420 including an object region to the second neural network model 430 to output a second segment 440. The first and second neural network models 410 and 430 may be models for detecting an area for lesion diagnosis from the image to segment the area.

When the object region for lesion diagnosis includes a lung and a lung lobe, a neural network model used to segment the lung area for lesion diagnosing and a neural network model used to segment the lung lobe area for lesion diagnosing are different in the computing device. For example, in the computing device, the neural network model used to segment the lung area for lesion diagnosis includes a 2D segmentation model and the neural network model used to segment the lung lobe area for lesion diagnosis may include a 3D segmentation model. However, this is merely an example, but the present disclosure is not limited thereto. The reason why different models are used to segment the lung and the lung lobe is because a 3D segmentation model which has a more computational amount than the 2D segmentation model is used only for the segmentation of the lung lobe to increase the computational efficiency and enable precise segmentation.

When at least one finding region related to a specific lesion is segmented from the medical data, the computing device segments at least one finding region related to a specific lesion, among a plurality of finding regions corresponding to ground glass opacity (GGO), consolidation, reticular opacity, pleural effusion, emphysema, and normal, from the medical data. When at least one finding region related to a specific lesion is segmented from the medical data, the computing device may segment a first finding region corresponding to the ground glass opacity (GGO) related to the corona virus disease including COVID-19, a second finding region corresponding to consolidation, a third finding region corresponding to reticular opacity, a fourth finding region corresponding to pleural effusion, and a fifth finding region corresponding to emphysema, from the medical data.

When at least one finding region related to a specific lesion is segmented from the medical data, the computing device segments at least one finding region related to a specific lesion from medical data using a second neural network model including a 2D segmentation model or a 3D segmentation model. For example, the 2D segmentation model may be a 2D U-Net, but this is merely an example, but the present disclosure is not limited thereto. Further, the 3D segmentation model may be a 3D U-Net, but this is merely an example, but the present disclosure is not limited thereto.

The computing device performs a quantification analysis 450 for the finding region included in the object region by calculating a volume and a location for at least one finding region included in the object region for lesion diagnosis. When a volume and a location for at least one finding region included in the object region for lesion diagnosis are calculated, the computing device calculates a volume for the object region and calculates a volume and a location for the finding region included in the obj ect region, and calculates a relative volume ratio of the finding region for the object region. When there is a plurality of finding regions, the computing device calculates a total volume for the plurality of finding regions and a relative total volume ratio of the plurality of finding regions with respect to the object region.

The computing device diagnoses and predicts the respiratory disease by inputting quantification data corresponding to the volume and the location for the finding region calculated through the quantification analysis 450 to the previously trained third neural network model 460. The third neural network model 460 may be a model which classifies and predicts a class for medical data. For example, the third neural network model 460 includes a random forest model. The third neural network model 460 may include a machine learning model including a neural network. The computing device diagnoses the respiratory disease from the medical data to generate and display the result information. When there is a plurality of object regions for lesion diagnosis, the computing device inputs the quantification data including a relative volume of each finding region for each object region, a distribution profile of each finding region, and a number of object regions in which the finding region is distributed based on a distribution profile of each finding region to the trained third neural network model 460 to diagnose and predict the respiratory disease. Further, the computing device inputs the quantification data corresponding to the volume and the location of the finding region to the trained third neural network model to classify the respiratory disease and normal. Further, the computing device inputs the quantification data corresponding to the volume and the location of the finding region to the trained third neural network model to classify the respiratory disease and the other disease. Further, the computing device inputs the quantification data corresponding to the volume and the location of the finding region to the trained third neural network model to classify a severe case and a mild case for the respiratory disease. Further, the computing device inputs the quantification data corresponding to the volume and the location of the finding region to the trained third neural network model to classify the treatment prognosis for the respiratory disease into a high risk group, a medium risk group, and a low risk group.

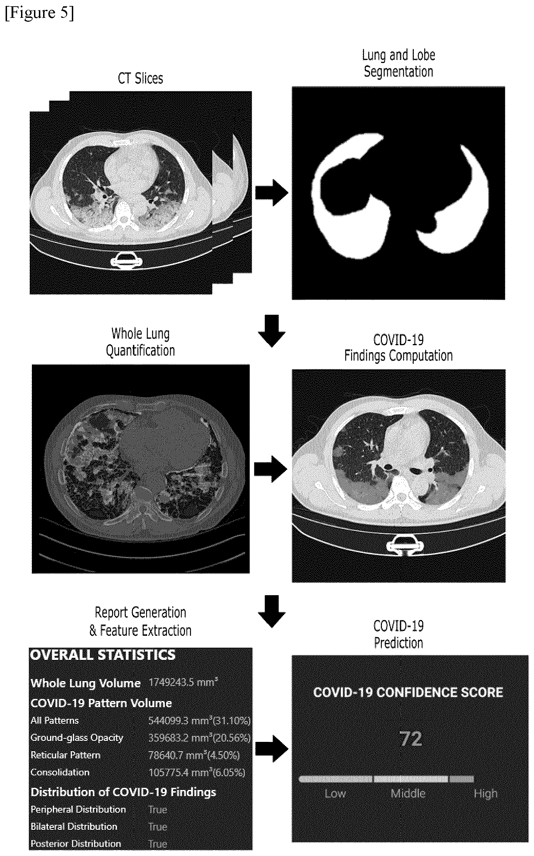

FIG. 5 is a view for explaining a process of diagnosing a corona virus disease including COVID-19, according to an exemplary embodiment of the present disclosure.

As illustrated in FIG. 5, the computing device of the present disclosure inputs the medical data including a plurality of CT slice images to the neural network model to segment the lung and the lung lobe for diagnosis of the corona virus disease including COVID-19. The computing device inputs the medical data including a plurality of CT slice images to the neural network model to segment the finding region for the corona virus disease related lesion. Next, the computing device calculates and quantifies a whole lung volume and calculates a volume and a location for the finding region related to the corona virus disease to perform quantification analysis on the finding region included in the lung area. The computing device calculates a volume for the lung area for lesion diagnosis, calculates a volume and a location for the finding region included in the lung area, and calculates a relative volume ratio of the finding region for the lung area. The computing device calculates and quantifies the volume for each lung lobe and calculates a volume and a location for the finding region related to the corona virus disease to perform quantification analysis on the finding region included in the lung lobe area. The computing device calculates a volume for the lung lobe area for lesion diagnosis, calculates a volume and a location for the finding region included in the lung lobe area, and calculates a relative volume ratio of the finding region for the lung lobe area. The computing device calculates a total volume for the plurality of finding regions and a relative total volume ratio of the plurality of finding regions with respect to the lung area.

The computing device inputs quantification data corresponding to the volume and the location for the finding region to the previously trained neural network model to diagnose and predict the corona virus disease including COVID-19. The computing device diagnoses the corona virus disease including COVID-19 from the medical data to generate and display the result information. The computing device provides a prediction probability score for the corona virus disease including COVID-19 based on the result information.

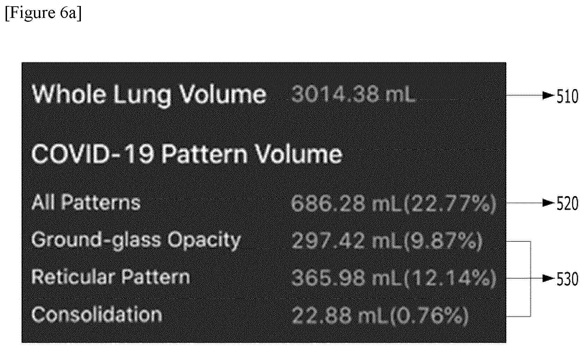

FIGS. 6a to 6c are views for explaining a process of quantifying an object region and a finding region for lesion diagnosis, according to an exemplary embodiment of the present disclosure.

As illustrated in FIG. 6a, the computing device of the present disclosure calculates a volume 510 for the whole lung and calculates a volume 530 for the finding region related to the corona virus disease including COVID-19 to perform quantification analysis for the finding region included in the lung area. That is, the computing device calculates a volume 510 for the whole lung area for lesion diagnosis, calculates a volume 530 for the finding region included in the lung area, and calculates a relative volume ratio of the finding region for the lung area. When there is a plurality of finding regions, the computing device calculates a total volume 520 for the plurality of finding regions and a relative total volume ratio of the plurality of finding regions with respect to the lung area. For example, the finding region includes a first finding region corresponding to the ground glass opacity (GGO) related to the corona virus disease lesion including COVID-19, a second finding region corresponding to consolidation, and a third finding region corresponding to reticular opacity.

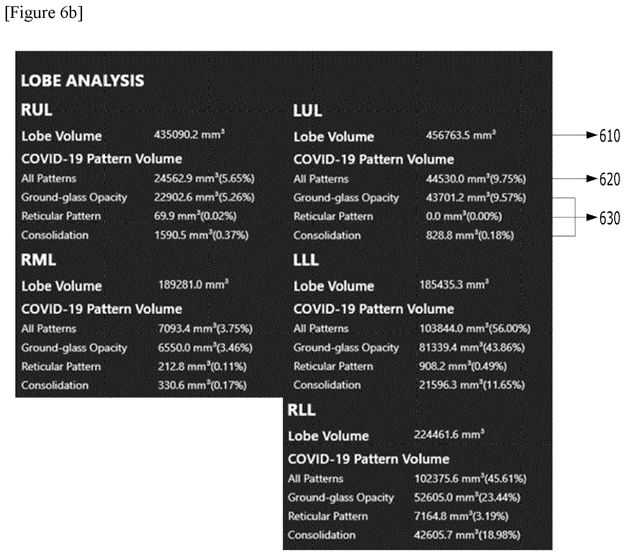

As illustrated in FIG. 6b, the computing device of the present disclosure calculates a volume 610 for each lung lobe and calculates a volume 630 for the finding region related to the corona virus disease including COVID-19 to perform quantification analysis for the finding region included in the lung lobe area. That is, the computing device calculates a volume 610 for each lung lobe area for lesion diagnosis, calculates a volume 630 for the finding region included in the lung lobe area, and calculates a relative volume ratio of the finding region for the lung lobe area. When there is a plurality of finding regions, the computing device calculates a total volume 620 for the plurality of finding regions and a relative total volume ratio of the plurality of finding regions with respect to the lung lobe area. For example, the plurality of lung lobe areas includes a right upper lung lobe (RUL), a right middle lung lobe (RML), a right lower lung lobe (RLL), a left upper lung lobe (LUL), and a left lower lung lobe (LLL). Further, the finding region includes a first finding region corresponding to the ground glass opacity (GGO) related to the corona virus disease lesion including COVID-19, a second finding region corresponding to consolidation, and a third finding region corresponding to reticular opacity. For example, the first finding region corresponding to the ground glass opacity (GGO) refers to an area which in the medical data which is a CT image, if there is partial filling of the alveolar cavity or slight thickening of the alveolar wall, does not appear as an independent object shade, but appear as a blurry shade. The second finding region corresponding to consolidation refers to an area which appears as an opaque shade when the lung is filled with exudate, pus, blood, or other cells or substances instead of air in the medical data which is a CT image. The third finding region corresponding to reticular opacity refers to an area which appears as reticular opacity when the tissues constituting the lung interstitium are thickened by liquid material, fibrotic tissue, or cells in the medical data which is a CT image. However, this is merely an example, but the present disclosure is not limited thereto.

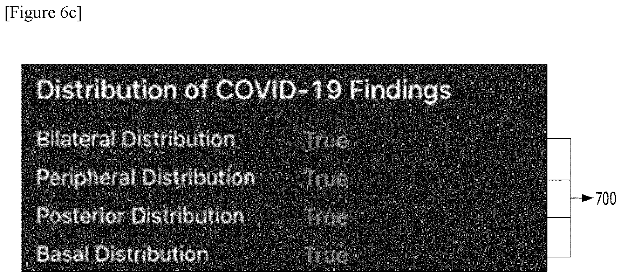

As illustrated in FIG. 6c, when a volume and a location for at least one finding region included in the lung and the lung lobe are calculated, the computing device of the present disclosure calculates a distribution profile 700 of the finding region based on the location of the finding region. For example, the distribution profile 700 of the finding region includes at least any one of bilateral distribution of the finding region, peripheral distribution, posterior distribution, and basal distribution.

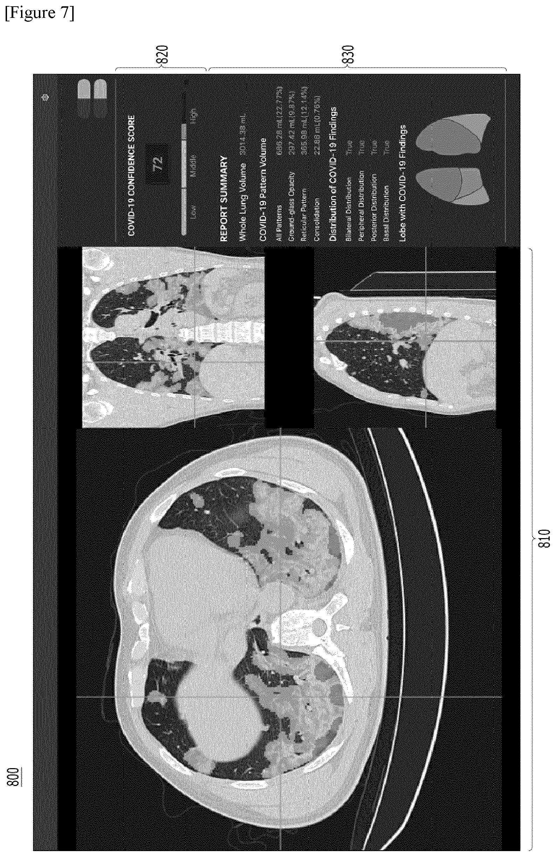

FIG. 7 is a view illustrating result information for medical data according to an exemplary embodiment of the present disclosure.

As illustrated in FIG. 7, the computing device of the present disclosure generates and displays result information 800 by diagnosing a corona virus disease including COVID-19 from the medical data.

The computing device of the present disclosure diagnoses the corona virus disease including COVID-19 from the medical data to extract and display the result information 800 from a database of an external server or an internal memory.

The result information 800 includes at least one of a distribution image 810 of the finding region included in the object region for lesion diagnosis, prediction probability information 820 for respiratory disease, and summary information 830 for the object region for lesion diagnosis and the finding region included in the object region.

The result information 800 may include a distribution image 810 of the finding region seen from at least any one of an upper direction, a lower direction, a left direction, a right direction, a front direction, and a rear direction of the object region for lesion diagnosis.

When there is a plurality of finding regions included in the object region for lesion diagnosis, as the result information 800, the plurality of finding regions includes a distribution image 810 of the finding regions with different colors.

The result information 800 includes a prediction probability information 820 for respiratory disease having at least one of a confidence score for the respiratory disease prediction and infographics.

The result information 800 includes summary information 830 for the finding region having at least one of volume information for the object region for lesion diagnosis, volume information for the finding region included in the object region, and distribution information for the finding region included in the object region. The volume information for the finding region includes relative volume information of the finding region with respect to the object region and relative volume ratio information. When there is a plurality of finding regions, the volume information for the finding region includes total volume information for the plurality of finding regions and relative total volume ratio information of the plurality of finding regions with respect to the object region. For example, the distribution information of the finding region includes at least any one of bilateral distribution of the finding region, peripheral distribution, posterior distribution, and basal distribution. The summary information for the finding region may include inforgraphics information in which a region which is predicted as the respiratory disease is visually segmented, among sub segmentation regions of the object region for lesion diagnosis based on predicted probability information of the respiratory disease. The summary information for the finding region may include inforgraphics information in which a sub segmentation region having a prediction probability of the respiratory disease which is higher than a reference probability is visually segmented, among sub segmentation regions of the object region for lesion diagnosis. When the object region for lesion diagnosis is a lung and sub segmented regions are a plurality of lung lobes, the summary information for the finding region may include inforgraphics information in which a lung lobe having a prediction probability of the respiratory disease which is higher than a reference probability is visually segmented, among the plurality of lung lobes.

The result information 800 may include at least any one of information for comparing the respiratory disease and the normal case, information for comparing the respiratory disease and the other disease, and information for comparing a severe case and a mild case for the respiratory disease.

As described above, the present disclosure may provide user's convenience by visualizing the finding region for the respiratory disease diagnosed from the medical data and quantitatively displaying the volume and the location of the finding region.

Further, the present disclosure assists in polymerase chain reaction (PCR) test, assists in physician diagnosis, or quantifies the severity and progression of a disease.

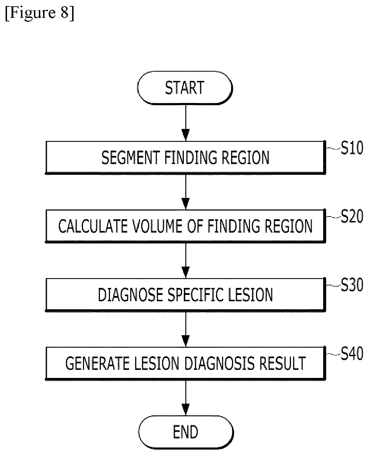

FIG. 8 is a flowchart for explaining a lesion diagnosis process according to an exemplary embodiment of the present disclosure.

As illustrated in FIG. 8, the computing device of the present disclosure segments an object region for lesion diagnosis and at least one finding region related to the respiratory disease from medical data by inputting the medical data to at least one neural network model (S10). The computing device segments at least object region for lesion diagnosis from medical data by inputting the medical data to a first neural network model and segments at least one finding region related to a specific lesion from the medical data by inputting the medical data to a second neural network model. The computing device segments at least object region for lesion diagnosis from medical data by inputting the medical data to a first neural network model and segments at least one finding region related to a specific lesion from the object region by inputting the medical data corresponding to the segmented object region to the second neural network model.

When at least the object region for lesion diagnosis is segmented from the medical data, if there is a plurality of object regions for lesion diagnosis, the computing device segments the object regions for lesion diagnosis using different neural network models. For example, when the object region for lesion diagnosis includes a lung and a lung lobe, a neural network model used to segment the lung area for lesion diagnosing and a neural network model used to segment the lung lobe area for lesion diagnosing are different in the computing device.

When at least one finding region related to the respiratory disease is segmented from the medical data, the computing device segments at least one finding region related to the respiratory disease from medical data using a second neural network model including a 2D segmentation model or a 3D segmentation model. When at least one finding region related to the respiratory disease is segmented from the medical data, the computing device segments an infected area related to the respiratory disease from the medical data using the 3D segmentation model and segments at least one finding region from the segmented infected area using the 2D segmentation model.

The computing device of the present disclosure calculates a volume and a location for at least one finding region included in at least object region for lesion diagnosis (S20). The computing device calculates a volume for the object region for lesion diagnosis, calculates a volume and a location for the finding region included in the object region for lesion diagnosis, and calculates a relative volume ratio of the finding region for the object region. When there is a plurality of finding regions, the computing device calculates a total volume for the plurality of finding regions and a relative total volume ratio of the plurality of finding regions with respect to the object region. The computing device may calculate a distribution profile of the finding region based on the location of the finding region.

Next, the computing device of the present disclosure diagnoses the respiratory disease based on the volume and the location for the finding region (S30). The computing device inputs the quantification data corresponding to the volume and the location of the finding region to the trained neural network model to diagnose and predict the respiratory disease. When there is a plurality of object regions for lesion diagnosis, the computing device inputs the quantification data including a relative volume of each finding region for each object region, a distribution profile of each finding region, and a number of object regions in which the finding region is distributed based on a distribution profile of each finding region to the trained neural network model to diagnose and predict the respiratory disease. Further, the computing device inputs the quantification data corresponding to the volume and the location of the finding region to the trained neural network model to classify the respiratory disease and normal. Further, the computing device inputs the quantification data corresponding to the volume and the location of the finding region to the trained neural network model to classify the respiratory disease and the other disease. Further, the computing device inputs the quantification data corresponding to the volume and the location of the finding region to the trained neural network model to classify a severe case and a mild case for the respiratory disease.

Next, the computing device of the present disclosure diagnoses the respiratory disease from the medical data to generate and display the result information (S40). The computing device generates and displays the result information including at least one of a distribution image of the finding region included in the object region for lesion diagnosis, prediction probability information for respiratory disease, and summary information for the object region for lesion diagnosis and the finding region included in the object region. For example, the result information includes summary information for the finding region having at least one of volume information for the object region for lesion diagnosis, volume information for the finding region included in the object region, and distribution information for the finding region included in the object region. The result information related to the lesion diagnosis may include at least any one of information for comparing the respiratory disease and the normal case, information for comparing the respiratory disease and the other disease, and information for comparing a severe case and a mild case for the respiratory disease.

Accordingly, according to the present disclosure, a respiratory disease is diagnosed from the medical data to increase a prediction probability for the respiratory disease. For example, a confidence for the diagnosis of the respiratory disease, such as corona virus including COVID-19 may be improved.

Further, the present disclosure may provide user's convenience by visualizing the finding region for the respiratory disease diagnosed from the medical data and quantitatively displaying the volume and the location of the finding region.

Further, the present disclosure assists in polymerase chain reaction (PCR) test, assists in physician diagnosis, or quantifies the severity and progression of a disease.

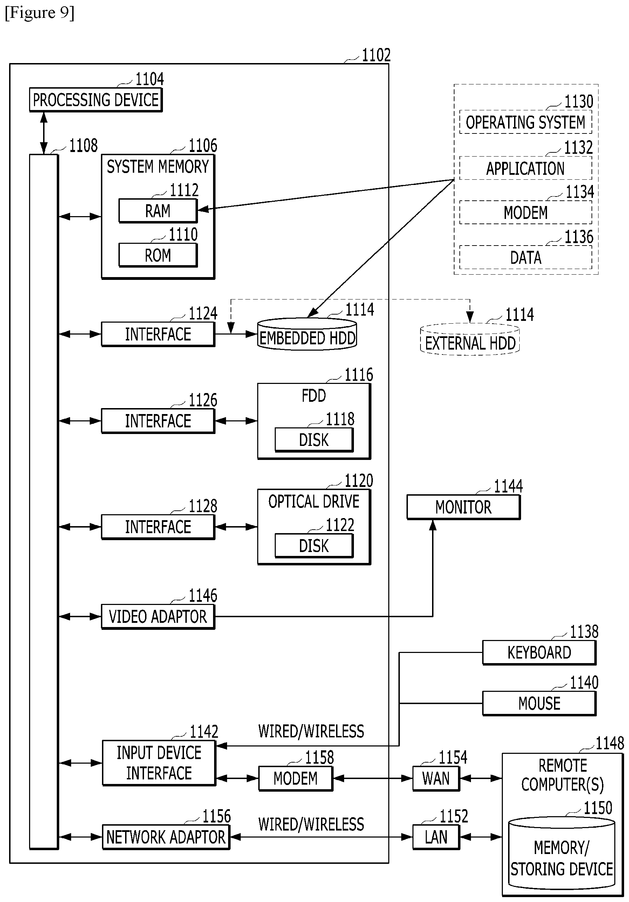

FIG. 9 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An exemplary environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined processor among various commercial processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802. 1 1a) or 54 Mbps (802. 1 1b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

### [Mode for invention]

Relevance contents in the best mode for carrying the present invention have been described as described above.

### [Industrial Applicability]

The present invention may be used for a computing device which provides a lesion diagnosis result using a neural network.

## Claims

1. A lesion diagnosing method, comprising:
inputting medical data into a first neural network model and a second neural network model to detect an object region for lesion diagnosis from the medical data, and to detect at least one finding region related to a specific lesion;
calculating a volume and a location for at least one finding region included in the object region; and
generating result information for the medical data based on the volume and the location for the finding region.

2. The lesion diagnosing method of claim 1, wherein the detecting of the object region and at least one finding region related to the specific lesion includes:
detecting the object region for lesion diagnosis from the medical data by inputting the medical data to the first neural network model; and
detecting at least one finding region related to the specific lesion from the medical data by inputting the medical data to the second neural network model.

3. The lesion diagnosing method of claim 1, wherein the detecting of the object region and at least one finding region related to the specific lesion includes:
detecting the object region for lesion diagnosis from the medical data by inputting the medical data to the first neural network model; and
detecting at least one finding region related to the specific lesion from the object region by inputting medical data including the object region detected through the first neural network model to the second neural network model.

4. The lesion diagnosing method of claim 1, wherein the detecting of at least one finding region related to the specific lesion from the medical data includes:
detecting a plurality of finding regions for lesions related to a respiratory disease from the medical data, and
the plurality of finding regions includes: a first finding region corresponding to ground glass opacity (GGO), a second finding region corresponding to consolidation, a third finding region corresponding to reticular opacity, a fourth finding region corresponding to pleural effusion, and a fifth finding region corresponding to emphysema.

5. The lesion diagnosing method of claim 1, wherein the calculating of the volume and the location for at least one finding region included in the object region includes:
calculating a volume for the object region;
calculating a volume and a location for a finding region included in the object region; and
calculating a relative volume ratio of the finding region to the object region.

6. The lesion diagnosing method of claim 5, further comprising:
when there is a plurality of finding regions, calculating a total volume for the plurality of finding regions and a relative total volume ratio of the plurality of finding regions with respect to the object region.

7. The lesion diagnosing method of claim 1, wherein the generating of result information for the medical data based on the volume and the location for the finding region includes:
generating result information for the medical data by inputting quantification data corresponding to the volume and the location for the finding region to a third neural network model.

8. The lesion diagnosing method of claim 1, wherein the generating of result information for the medical data based on the volume and the location for the finding region includes:
classifying a class for the medical data by inputting quantification data corresponding to the volume and the location for the finding region to a third neural network model.

9. The lesion diagnosing method of claim 8, wherein the class represents a class of the medical data related to a respiratory disease, and the class includes at least one of: normal, abnormal, a mild case, a severe case, or a low risk group, a medium risk group, a high risk group corresponding to a treatment prognosis, or a type of a respiratory disease.

10. The lesion diagnosing method of claim 1, wherein in the generating of result information for the medical data based on the volume and the location for the finding region includes:
calculating a respiratory disease prediction probability score included in the medical data based on a location, an absolute volume, and a relative volume of each finding region for a lung volume, and a location, an absolute volume, and a relative volume of each finding region for each lung lobe volume, when there is a plurality of finding regions, and
wherein each finding region is any one of: a first finding region corresponding to ground glass opacity (GGO), a second finding region corresponding to consolidation, a third finding region corresponding to reticular opacity, a fourth finding region corresponding to pleural effusion, and a fifth finding region corresponding to emphysema.

11. A user terminal for lesion diagnosis, comprising:
a processor including one or more cores;
a memory; and
an output unit for providing a user interface,
wherein the user interface displays result information for medical data in response to medical data input, and
wherein the result information for the medical data is generated based on a result of calculating a volume and a location for at least one finding region included in an object region, based on the at least one finding region related to a specific lesion and the object region for lesion diagnosis detected from the medical data.

12. The user terminal for lesion diagnosis of claim 11, wherein the result information for the medical data includes at least one of: summary information for the object region for lesion diagnosis and the finding region included in the object region, prediction probability information for respiratory disease, and a distribution image of the finding region included in the object region for lesion diagnosis.

13. The user terminal for lesion diagnosis of claim 11, wherein the user interface displays result information for the medical data in response to a user input, and the result information for the medical data is extracted from a database in which result information generated based on the volume and the location for at least one finding region included in the object region is stored.

14. A computing device for providing a lesion diagnosing method, comprising:
a processor including one or more cores; and
a memory,
wherein the processor is configured to:
input medical data into a first neural network model and a second neural network model to detect an object region for lesion diagnosis from the medical data, and to detect at least one finding region related to a specific lesion,
calculate a volume and a location for at least one finding region included in the object region, and
generate result information for the medical data based on a volume and a location for the finding region.
